(19) Europäisches Patentamt
European Patent Office
Office européen des brevets

(11) Veröffentlichungsnummer: **0 101 926**
**B1**

(12) # EUROPÄISCHE PATENTSCHRIFT

(45) Veröffentlichungstag der Patentschrift: **03.09.86**

(51) Int. Cl.⁴: **C 07 D 249/08**, C 07 D 233/61 // C07C49/163

(21) Anmeldenummer: **83107307.7**

(22) Anmeldetag: **26.07.83**

(54) **Azolyl-ketoenamine.**

(30) Priorität: **05.08.82 DE 3229275**

(43) Veröffentlichungstag der Anmeldung: **07.03.84 Patentblatt 84/10**

(45) Bekanntmachung des Hinweises auf die Patenterteilung: **03.09.86 Patentblatt 86/36**

(84) Benannte Vertragsstaaten: **BE CH DE FR GB IT LI NL**

(56) Entgegenhaltungen:
**EP-A-0 040 366**
**EP-A-0 040 367**
**EP-A-0 040 368**
**EP-A-0 041 615**
**EP-A-0 048 827**
**EP-A-0 101 925**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Elbe, Hans- Ludwig, Dr., Dasnöckel 59, D-5600 Wuppertal 11 (DE)**

EP 0 101 926 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft neue Azolyl-ketoenamine, ein Verfahren zu deren Herstellung und ihre Verwendung als Zwischenprodukte zur Synthese von Azolylvinyl-ketonen und -carbinolen, welche fungizide und pflanzenwuchsregulierende Eigenschaften besitzen.

Es ist bereits bekannt geworden, daß bestimmte 1-Vinyl-triazolyl-(halogen)-tert.-butyl-ketone bzw. -carbinole gute fungizide Eigenschaften besitzen, (vergleiche BE- A 29 06 06, DE-A-29 33 422, DE-A- 30 19 046 bzw. EP-A-0 040 363, DE-A-28 38 847 sowie die Japanische Patentanmeldung JP 531 30 661). Die Wirkung dieser Verbindungen ist jedoch, insbesondere bei niedrigen Aufwandmengen und -konzentrationen, nicht immer voll befriedigend.

Weiterhin sind bereits bestimmte Ketoenamine und deren Verwendung als Zwischenprodukte zur Herstellung von Stoffen mit fungizider bzw. antimikrobieller Wirksamkeit bekannt (vergleiche EP-A-0 040 336, EP-A-0 040 367, EP-A-0 040 368 und EP-A-0 048 827). Die betreffenden Ketoenamine enthalten als charakteristische Struktureinheit eine Gruppierung der Formel

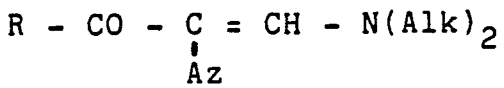

in welcher
Alk für Alkyl steht,
Az für 1,2,4-Triazol-1-yl oder Imidazol-1-yl steht
und
R für Alkyl, Halogenalkyl oder für durch bestimmte Reste substituiertes Phenyl steht.

Entsprechend Ketoenamine, in denen R für andere als die oben angegebenen Reste steht, sind jedoch nicht bekannt.

Es wurden nun neue Azolyl-ketoenamine der allgemeinen Formel

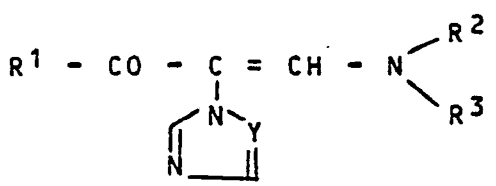
(I)

in welcher stehen
$R^1$ für geradkettiges oder verzweigtes Alkyl mit 1 bis 10 Kohlenstoffatomen; für geradkettiges oder verzweigtes Halogenalkyl mit 1 bis 4 Kohlenstoffund 1 bis 5 gleichen oder verschiedenen Halogenatomen, wie Fluor-, Chlor- und Bromatomen; für gegebenenfalls einfach oder mehrfach, gleich oder verschieden substituiertes Phenyl, wobei als Substituenten genannt seien: Halogen,
Alkyl mit 1 bis 4 Kohlenstoffatomen, Alkoxy und Alkylthio mit jeweils 1 bis 4 Kohlenstoffatomen, Alkyl und Dialkylamino mit jeweils 1 bis 4 Kohlenstoffatomen in jedem Alkylteil, ferner Halogenalkyl, Halogenalkoxy und Halogenalkylthio mit jeweils 1 bis 4 Kohlenstoffatomen und 1 bis 5 gleichen oder verschiedenen Halogenatomen, wie Fluor- und Chloratomen, sowie jeweils gegebenenfalls durch Halogen und Alkyl mit 1 bis 2 Kohlenstoffatomen substituiertes Phenyl und Phenoxy; weiterhin für gegebenenfalls einfach oder mehrfach, gleich oder verschieden durch Alkyl mit 1 bis 4 Kohlenstoffatomen und Halogen substituiertes Cycloalkyl mit 3 bis 7 Kohlenstoffatomen sowie für die Gruppierung

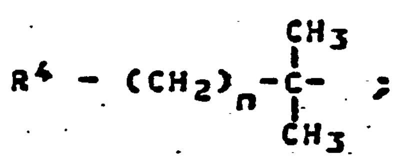

$R^4$ für gegebenenfalls einfach oder mehrfach, gleich oder verschieden substituiertes Phenyl, wobei als Substituenten die bei $R^1$ bereits genannten Phenylsubstituenten genannt seien, ferner für geradkettiges oder verzweigtes Alkenyl mit 2 bis 4 Kohlenstoffatomen und Alkinyl mit 3 bis 5 Kohlenstoffatomen, für Cyano sowie

für die Gruppierung -Z-$R^5$;

$R^5$ für geradkettiges oder verzweigtes Alkyl mit 1 bis 6 Kohlenstoffatomen; Halogenalkyl mit 1 bis 4 Kohlenstoffatomen und 1 bis 5 gleichen oder verschiedenen Halogenatomen, wie Fluor- und Chloratomen; sowie für jeweils gegebenenfalls einfach oder mehrfach, gleich oder verschieden substituiertes Phenyl und Phenylalkyl mit 1 bis 2 Kohlenstoffatomen im Allylteil, wobei jeweils als Phenylsubstituenten die bei $R^1$ bereits genannten Phenylsubstituenten infragekommen;

Z für O, S, SO oder $SO_2$,

n für die Zahlen 0 bis 2,

$R^2$ und $R^3$ gemeinsam mit dem N-Atom, an das sie gebunden sind, für jeweils gegebenenfalls einfach bis dreifach, gleich oder verschieden durch Alkyl mit 1 bis 4 Kohlenstoffatomen substituiertes Piperidinyl, Pyrrolidinyl oder Morpholinyl; oder

$R^2$ und $R^3$ sind gleich oder verschieden und stehen auch für Alkyl mit 1 bis 4 Kohlenstoffatomen, wenn $R^1$ für gegebenenfalls substituiertes Cycloalkyl oder die Gruppierung $R^4$-$(CH_2)_n$-$C(CH_3)_2$-steht; und

Y für ein Stickstoffatom oder die CH-Gruppe gefunden.

Weiterhin wurde gefunden, daß man die neuen Azolyl-ketoenamine der Formel (I) erhält, indem man Azolylketone der Formel

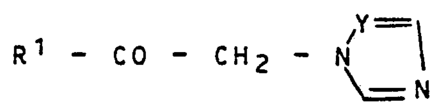

(II)

in welcher

$R^1$ und Y die oben angegebene Bedeutung haben,

mit Amidacetalen bzw. Aminalestern der Formeln

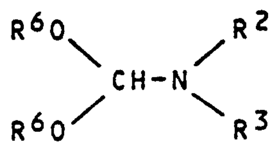

(IIIa)

bzw.

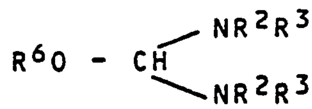

(IIIb)

in welcher

$R^2$ und $R^3$ die oben angegebene Bedeutung haben

und

$R^6$ für Alkyl mit 1 bis 4 Kohlenstoffatomen steht,

in Gegenwart eines Verdünnungsmittels umsetzt.

Die neuen Azolyl-ketoenamine sind interessante Zwischenprodukte zur Herstellung von Pflanzenschutz-Wirkstoffen. So eignen sich die Stoffe der Formel (I) als Ausgangsprodukte zur Synthese von Azolylvinyl-ketonen und -carbinolen, welche sehr gute fungizide und pflanzenwuchsregulierende Wirksamkeit besitzen.

Ueberraschenderweise sind die Azolylvinyl-ketone und -carbinole, die sich aus dem erfindungsgemäßen Azolyl-ketoenaminen der Formel (I) durch Umsetzung mit magnesium-organischen Verbindungen und gegebenenfalls anschließende Reduktion der Keto-Gruppe herstellen lassen, den aus dem Stand der Technik bekannten 1-Vinyltriazolyl-(halogen)-tert.-butyl-ketonen bzw. -carbinolen bezüglich ihrer fungiziden Wirksamkeit überlegen. Darüber hinaus zeichnen sich die aus den erfindungsgemäßen Stoffen der Formel (I) herstellbaren Azolylvinyl-ketone und -carbinole auch durch sehr gute pflanzenwuchsregulierende Eigenschaften aus.

Die erfindungsgemäßen Stoffe sind durch die Formel (I) allgemeinen definiert. Sie unterscheiden sich von den konstitutionell ähnlichen Verbindungen die aus der EP-A-0 040 366, der EP-A- 0 040 367, der EP-A-0 040 368 und der EP-A 0 048 827 bekannt sind, in charakteristischer Weise durch die Substituenten, die am Stickstoffatom bzw. an der Ketogruppe enthalten sind.

Bevorzugt sind diejenigen Verbindungen der Formel (I), in denen

$R^1$ für geradkettiges oder verzweigtes Alkyl mit 4 bis 7 Kohlenstoffatomen steht; für Chlor-tirt.-butyl, Fluor-tert.-butyl, 1,3-Dichlor-2-methyl-2-propyl und 1,3-Difluor-2-methyl-2-propyl steht; ferner für gegebenenfalls einfach bis dreifach, gleich oder verschieden substituiertes Phenyl steht, wobei als Substituenten genannt seien: Fluor, Chlor, Methyl, Isopropyl, tert.-Butyl, Methoxy, Methylthio, Isopropoxy, Trifluormethyl, Trifluormethoxy, Trifluormethylthio, Methylamino, Dimethylamino, gegebenenfalls durch Fluor, Chlor, Methyl substituiertes Phenyl und Phenoxy; außerdem für jeweils gegebenenfalls einfach bis dreifach, gleich oder verschieden durch Methyl, Ethyl, Isopropyl, tert.-Butyl, Chlor oder Brom substituiertes Cyclopropyl, Cyclopentyl und Cyclohexyl steht; sowie für die Gruppierung $R^4$-$(CH_2)_n$-

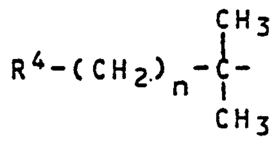 steht;

steht;

$R^4$ für gegebenenfalls einfach bis dreifach, gleich oder verschieden substituiertes Phenyl steht, wobei als Substituenten die bei $R^1$ bereits genannten Phenylsubstituenten genannt seien; ferner für Vinyl,Propargyl,Cyano sowie die Gruppierung -Z-$R^5$ steht;

$R^5$ für geradkettiges oder verzweigtes Alkyl mit 1 bis 4 Kohlenstoffatomen steht, sowie für jeweils gegebenenfalls einfach bis dreifach, gleich oder verschieden substituiertes Phenyl und Benzyl steht, wobei jeweils als Phenylsubstituenten die bei $R^1$ bereits genannten Phenylsubstituenten infragekommen;

$R^2$ und $R^3$ gemeinsam mit dem N-Atom, an das sie gebunden sind, für jeweils gegebenenfalls einfach bis dreifach, gleich oder verschieden durch Methyl, Ethyl und Isopropyl substituiertes Piperidinyl, Pyrrolidinyl oder Morpholinyl stehen; oder

$R^2$ und $R^3$ gleich oder verschieden sind und für Methyl, Ethyl, $_n$-Propyl i-Propyl oder n-Butyl stehen, wenn $R^1$ für gegebenenfalls substituirtes Cycloalkyl oder die Gruppierung $R^4$-$(CH_2)_n$-$C(CH_5)_2$-steht,

Y, Z und der Index n für die in der Erfindungsdefinition. angegebenen Bedeutungen stehen.

Verwendet man beispielsweise 1-(4-Chlorphenyl)-Z,Z-di-methyl-4-(1,2,4-triazol-1-yl)-3-butanon und Dimethylformamid-dimethylacetal als Ausgangsstoffe, so kann der Reaktionsablauf des erfindungsgemäßen Verfahrens durüh das folgende Formelschema wiedergegeben werden:

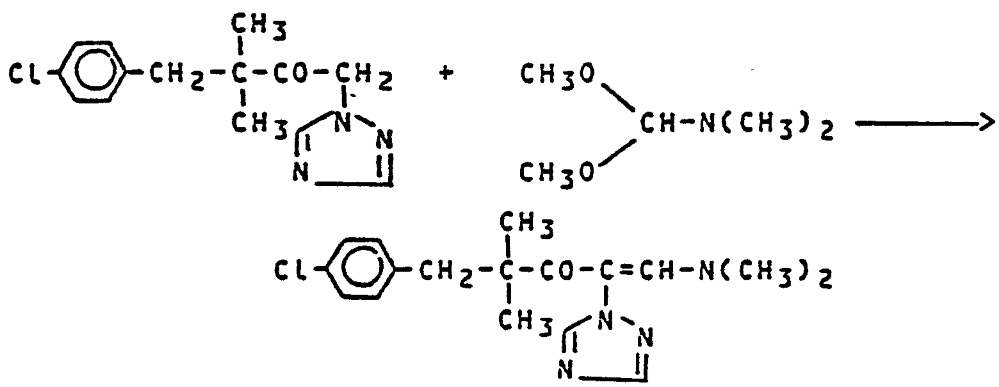

Die für das erfindungsgemäße Verfahren als Ausgangsstoffe zu verwendenden Azolylketone sind durch die Formel (II) allgemein definiert. In dieser Formel stehrn $R^1$ und Y vorzugsweise für diejenigen Bedeutungen, die bereits im Stoffe der Formel (I) vorzugsweise für diese Substituenten genannt wurden.

Die Azolylketone der Formel (II) sind bekannt (vergleiche DE-A-26 10 022, DE-A-26 38 470, DE-A-24 31 407, DE-A-29 06 061,

DE-A-30 28 330, DE-A-30 48 266, sowie die EP-A-0 080 096 und die EP-A-0 081 675 bzw. können sie nach üblichen Methoden hergestellt werden, indem man die entsprechenden Halogen-ketone in Gegenwart eines Säurebinders, wie beispielsweise Kaliumcarbonat, mit 1,2,4-Triazol oder Imidazol umsetzt.

Die außerdem für das erfindungsgemäße Verfahren als Ausgangsstoffe zu verwendenden Amidacetale bzw. Aminalester sind durch die Formeln (IIIa) bzw. (IIIb) allgemein definiert. In diesen Formeln stehen $R^2$ und $R^3$ für diejenigen Bedeutungen, die bereits im Zusammenhang mit der Beschreibung der erfindungsgemäßen Stoffe der Formel (1) als bevorzugt für diese Substituenten genannt wurden $R^6$ steht vorzugsweise für Methyl, Ethyl, n-Propyl, i-Propyl oder n-Butyl.

Die Amidacetale bzw. Aminalester der Formeln (IIIa) bzw. (IIIb) sind allgemein bekannte Verbindungen der

organischen Chemie (vergleiche z.B. Chem.Ber. 101, 41-50(1968) und J.Org.Chem. 43, 4248-50 (1978)); bzw. können sie nach den dort angegebenen Verfahren erhalten werden.

Für das erfindungsgemäße Verfahren kommen als Verdünnungsmittel inerte organische Lösungsmittel infrage. Hierzu gehören vorzugsweise aromatische Kohlenwasserstoffe, wie Benzol, Toluol oder Xylol; Formamide und Sulfoxide, wie Dimethylformamid und Dimethylsulfoxid; sowie insbesondere ein Ueberschuß an eingesetztem Amidacetal bzw. Aminalester der Formeln (IIIa) bzw. (IIIb).

Die Reaktionstemperaturen können bei dem erfindungsgemäßen Verfahren in einem größeren Bereich variiert werden. Im allgemeinen arbeitet man zwischen 40 und 180°C, vorzugsweise in der Siedehitze des verwendeten Lösungsmittels.

Bei der Durchführung des erfindungsgemäßen Verfahrens setzt man auf 1 Mol der Verbindungen der Formel (II) 1 bis 3 Mol Amidacetal bzw. Aminalester der Formel (IIIa) bzw. (IIIb) ein. Die Isolierung der Verbindungen der Formel (I) erfolgt in üblicher Art und Weise.

Die erfindungsgemäßen Azolyl-ketoenamine der Formel (I) eignen sich als Zwischenprodukte zur Synthese von Azolylvinyl-ketonen und -carbinolen, welche fungizide und pflanzenwuchsregulierende Wirksamkeit besitzen (vergleiche EP-A-0 101 925).

Solche Azolylvinyl-ketone und -carbinole der Formel

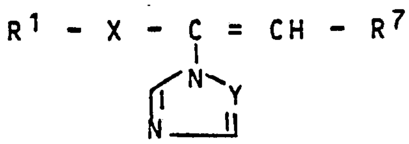

(IV)

in welcher
$R^1$ und Y die oben angegebene Bedeutung haben,
X für die CO- oder CH(OH)-Gruppe steht
und
$R^7$ für Alkyl, Alkenyl, Alkinyl, jeweils gegebenenfalls substituiertes Phenyl und Phenylalkyl, sowie für jeweils gegebenenfalls substituiertes Cycloalkyl, Cycloalkylalkyl, Cycloalkenyl, Cycloalkenylalkyl, Bicycloalkyl oder Bicycloalkenyl steht,
lassen sich herstellen, indem man Azolyl-ketoenamine der Formel

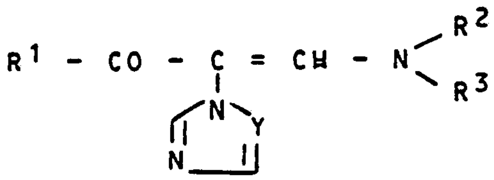

(I)

in welcher
$R^1$, $R^2$, $R^3$ und Y die oben angegebene Bedeutung haben,
mit magnesium-organischen Verbindungen der Formel [illegible] - Mg - $R^7$ (V)

$$Hal - Mg - R^7 \qquad (V)$$

in welcher
$R^7$ die oben angegebene Bedeutung hat und [illegible] für Halogen steht,
in Gegenwart eines Verdünnungsmittels umsetzt; und gegebenenfalls die so erhaltenen Azolylvinyl-ketone der Formel

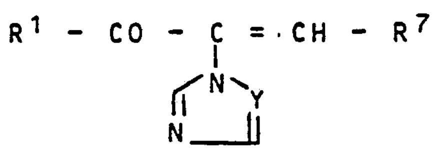

(IVa)

in welcher
$R^1$, $R^7$ und Y die oben angegebene Bedeutung haben,
in üblicher Weise reduziert.

Die bei der Umsetzung als Reaktionskomponente zu verwendenden magnesium-organischen Verbindungen sind durch die Formel (V) allgemein definiert. In dieser Formel steht Hal vorzugsweise für Chlor oder Brom. $R^7$ steht vorzugsweise für geradkettiges oder verzweigtes Alkyl mit 1 bis 12 Kohlenstoffatomen; geradkettiges oder verzweigtes Alkenyl und Alkinyl mit jeweils 2 bis 7 Kohlenstoffatomen; für jeweils gegebenenfalls einfach oder mehrfach, gleich oder verschieden substituiertes Phenyl und Phenylalkyl mit 1 bis 4 Kohlenstoffatomen im

Alkylteil, wobei jeweils als Phenylsubstituenten vorzugsweise die bei $R^3$ bereits genannten Phenylsubstituenten infragekommen; weiterhin fur jeweils gegebenenfalls einfach oder mehrfach, gleich oder verschieden durch Alkyl mit 1 bis 4 Kohlenstoffatomen und Halogen substituiertes Cycloalkyl mit 3 bis 12 Kohlenstoffatomen, Cycloalkylalkyl mit 3 bis 7 Kohlenstoffatomen im Cycloalkylteil und 1 bis 4 Kohlenstoffatomen im Alkylteil, Cycloalkenyl mit 3 bis 12 Kohlenstoffatomen, Cycloalkenylalkyl mit 3 bis 7 Kohlenstoffatomen im Cycloalkenylteil und 1 bis 4 Kohlenstoffatomen im Alkylteil, Bicycloalkyl mit 4 bis 24 Kohlenstoffatomen oder Bicycloalkenyl mit 4 bis 24 Kohlenstoffatomen.

Die magnesium-organischen Verbindungen der Formel (V) sind allgemein bekannte Verbindungen der organischen Chemie.

Für die Umsetzung der erfindungsgemäßen Azolyl-ketoenamine der Formel (I) mit magnesium-organischen Verbindungen der Formel (V) kommen als Verdünnungsmittel vorzugsweise inerte organische Lösungsmittel in reiner Form oder im Gemisch infrage. Hierzu gehören vorzugsweise Ether, wie Diethylether, Methylethylether, Tetrahydrofuran oder Dioxan, aliphatische und aromatische Kohlenwasserstoffe, wie insbesondere Benzol, Toluol oder Xylol, sowie Hexamethylphosphorsäuretriamid.

Die Reaktionstemperaturen können bei den angegebenen Verfahren zur Herstellung von Azolylvinyl-ketonen (IVa) in einem größeren Bereich variiert werden. Im allgemeinen arbeitet man zwischen -50 und + 150°C, vorzugsweise zwischen -20 und +120°C.

Bei der Durchführung des oben angegebenenVerfahrens zur Herstellung von Azolylvinyl-ketonen (IVa) setzt man auf 1 Mol Azolyl-ketoenamin der Formel (I) vorzugsweise 1 bis 1,5 Mol an magnesium-organischer Verbindung der Formel (V) ein.

Die Isolierung der Endprodukte erfolgt in üblicher Weise.

Die oben angegebene Reduktion der Azolyl-ketone der Formel (IV a) erfolgt in üblicher Art und Weise, z.B. durch Umsetzung mit komplexen Hydriden, gegebenenfalls in Gegenwart eines Verdünnungsmittels, oder durch Umsetzung mit Aluminiumisopropylat in Gegenwart eines Verdünnungsmittels.

Arbeitet man mit komplexen Hydriden, so kommen als Verdünnungsmittel für diese Umsetzung polare organische Lösungsmittel infrage. Hierzu gehören vorzugsweise Alkohole, wie Methanol, Ethanol, Butanol, Isopropanol und-Ether, wie Diethylether oder Tetrahydrofuran. Die Reaktion wird im allgemeinen bei 0 bis 30°C, vorzugsweise bei 0 bis 20°C-durchgeführt. Hierzu setzt man auf 1 Mol des Ketons der Formel (IVa) etwa 1 Reaktionsäquivalent eines komplexen hydrids, wie Natriumborhydrid oder Lithiumalanat, ein. Zur Isolierung der reduzierten Verbindungen der Formel (IV) wird der Rückstand in verdünnter Salzsäure aufgenommen, anschließend alkalisch gestellt und mit einem organischen Lösungsmittel extrahiert. Die weitere Aufarbeitung erfolgt in üblicher Weise.

Arbeitet man mit Aluminiumisopropylat, so kommen als Verdünnungsmittel für diese Umsetzung bevorzugt Alkohole, wie Isopropanol, oder inerte Kohlenwasserstoffe, wie Benzol, infrage. Die Reaktionstemperaturen können wiederum in einem größeren Bereich variiert werden; im allgemeinen arbeitet man zwischen 20 und 120°C, vorzugsweise bei 50 bis 100°C. Zur Durchführung der Reaktion setzt man auf 1 Mol des Ketons der Formel (IVa) etwa 1 bis 2 Mol Aluminiumisopropylat ein. Zur Isolierung der reduzierten Verbindungen der Formel (IV) wird das überschüssige Lösungsmittel durch Destillation im Vakuum entfernt und die entstehende Aluminiumverbindung mit verdünnter Schwefelsäure oder Natronlauge zersetzt. Die weitere Aufarbeitung erfolgt in üblicher Weise.

Die aus den erfindungsgemäßen Stoffen herstellbaren Azolylvinyl-ketone und -carbinole der Formel (IV) besitzen sehr gute fungizide und pflanzenwuchsregulierende Eigenschaften. Insbesondere können sie zur Bekämpfung solcher Pilze eingesetzt werden, die Getreidekrankheiten hervorrufen (vergleiche EP-A-0 101 925).

**Herstellungsbeispiele**

**Beispiel 1**

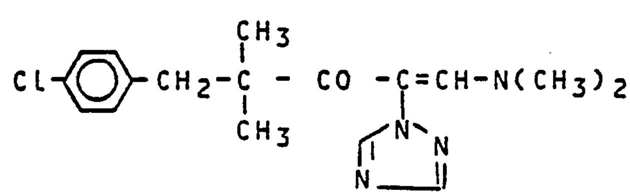

50g (0,18 Mol) 1-(4-Chlorphenyl)-2,2-dimethyl-4-(1,2,4-triazol-1-yl)-3-butanon werden mit 23,6g (0,138 Mol) Dimethylformamid-dimethylacetal 8 Stunden unter Rückfluß erhitzt. Zur Isolierung des Endproduktes wird das Reaktionsgemisch im Vakuum eingeengt. Man erhält 56,5g (94,4 % der Theorie) 1-(4-Chlorphenyl)-2,2-dimethyl-5-dimethyl-amino-4-(1,2,4-triazol-1-yl)-4-penten-3-on vom Brechungsindex $n_D^{20} = 1,5797$.

Herstellung des Ausgangsproduktes

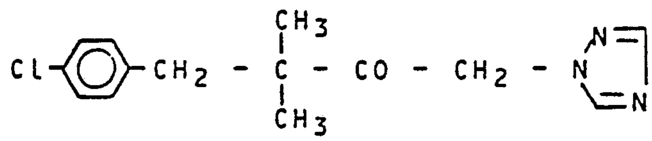

37g (0,13 Mol) 4-Brom-1-(4-chlorphenyl)-2,2-dimethyl-3-butanon, 13,3g (0,019 Mol) 1,2,4-Triazol und 53,8g (0,39 Mol) Kaliumcarbonat werden in 300 ml Aceton 8 Stunden unter Rückfluß erhitzt. Man läßt abkühlen, saugt vom anorganischen Rückstand ab und engt das Filtrat ein. Der Rückstand wird in Chloroform aufgenommen mit Wasser gewaschen, über Natriumsulfat getrocknet und eingeengt. Der Rückstand wird mit Diethylether verrührt, abgesaugt und bei 50°C im Vakuum getrocknet. Man erhält 18,8g (52 % der Theorie) 1-(4-Chlorphenyl)-2,2-dimethyl-4-(1,2,4-triazol-1-yl)-3-butanon vom Schmelzpunkt 127°C.

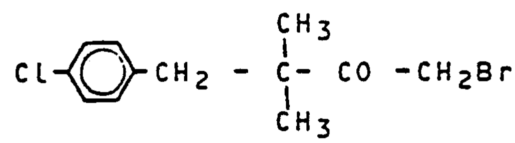

130g (0,62 Mol) 1-(4-Chlorphenyl)-2,2-dimethyl-3-butanon in 1000 ml Chloroform werden bei Raumtemperatur tropfenweise mit 98,8g (0,62 Mol) Brom versetzt. Man läßt das Reaktionsgemisch 1 Stunde nachrühren und engt anschließend ein. Man erhält 174,7g (97,3 % der Theorie) 4-Brom-1-(4-chlorphenyl)-2,2-dimethyl-3-butanon vom Brechungsindex $n^{20}_{D} = 1,5570$.

**Beispiel 2**

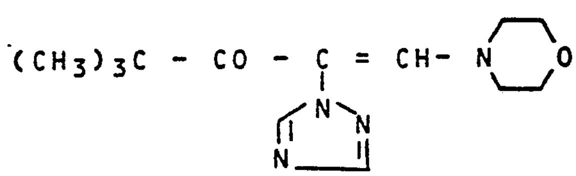

8,4g (0,05 Mol) 1,2,4-Triazol-1-yl-pinakolin werden mit 16,1g (0,1 Mol) N-Dimethoxymethyl-morpholin 9 Stunden bei 140°C gerührt. Man läßt das Reaktionsgemisch abkühlen und versetzt mit einem Gemisch aus Diisopropylether/Hexan (1:1), bis das Endprodukt vollständig ausgefallen ist. Der kristalline Niederschlag wird abgetrennt und bei 50°C im Vakuum getrocknet. Man erhält 11,3g (85,6 % der Theorie) 2,2-Dimethyl-5-morpholin-1-yl-4-(1,2,4-triazol-1-yl)-4-pen-ten-3-on vom Schmelzpunkt 125°C.

In analoger Weise und entsprechend dem erfindungsgemäßen Verfahren werden die nachstehend aufgeführten Verbindungen der Formel

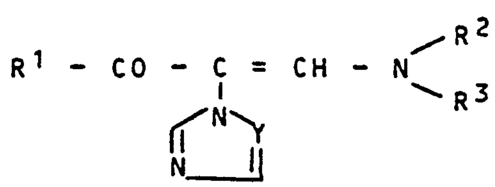

(I)

erhalten:

| Bsp. Nr. | $R^1$ | Y | 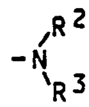 | Schmelzpunkt (°C) bzw. $n_D^{20}$ |
|---|---|---|---|---|
| 3 | 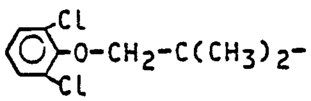 | N | $-N(CH_3)_2$ | zähes Oel |
| 4 | 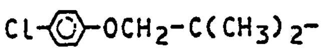 | N | $-N(CH_3)_2$ | 10 |
| 5 | 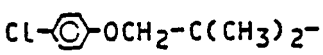 | CH | $-N(CH_3)_2$ | zähes Oel |
| 6 | 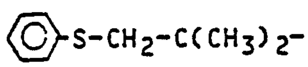 | N | $-N(CH_3)_2$ | 76-78 |
| 7 | 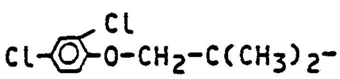 | CH | $-N(CH_3)_2$ | 1,5511 |
| 8 | 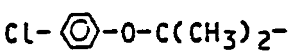 | N | $-N(CH_3)_2$ | 132 |
| 9 | $C_2H_5-OCH_2-C(CH_3)_2-$ | N | $-N(CH_3)_2$ | 1,4868 |
| 10 | $CH_3-OCH_2-C(CH_3)_2-$ | N | $-N(CH_3)_2$ | 1,5285 |
| 11 | 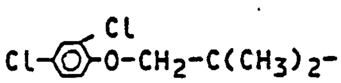 | N | $-N(CH_3)_2$ | 1,5723 |
| 12 | 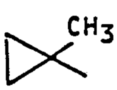 | N | $-N(CH_3)_2$ | 1,5695 |
| 13 | $(CH_3)_3C-$ | N | 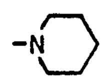 | 1,5419 |

| Bsp. Nr. | $R^1$ | Y | $-NR^2R^3$ | Schmelzpunkt (°C) bzw. $n_D^{20}$ |
|---|---|---|---|---|
| 14 | 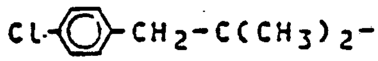 | N | 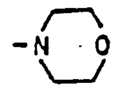 | 1,5699 |
| 15 | 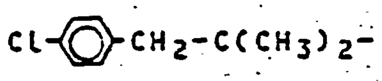 | N | 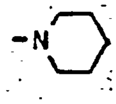 | 1,5480 |
| 16 | 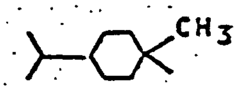 | CH | $-N(CH_3)_2$ | 88 |
| 17 | 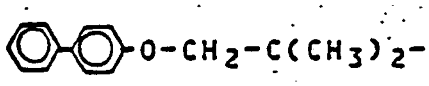 | CH | $-N(CH_3)_2$ | 126 |
| 18 | 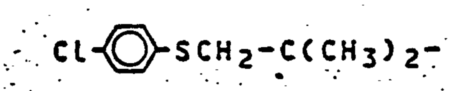 | CH | $-N(CH_3)_2$ | zähes Oel |
| 19 | 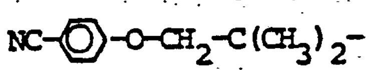 | CH | $-N(CH_3)_2$ | 1,5613 |
| 20 | 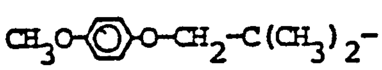 | CH | $-N(CH_3)_2$ | 1,5661 |
| 21 | 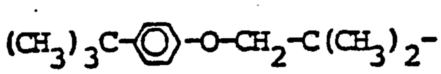 | CH | $-N(CH_3)_2$ | 1,5571 |
| 22 | 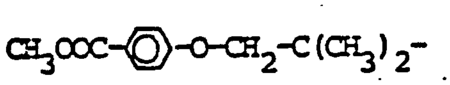 | CH | $-N(CH_3)_2$ | 1,5706 |
| 23 | 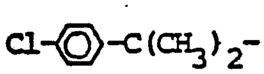 | CH | $-N(CH_3)_2$ | 176 |
| 24 | 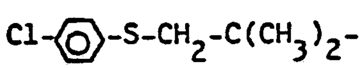 | N | $-N(CH_3)_2$ | öl |
| 25 | 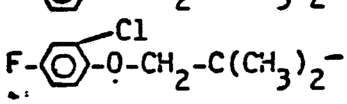 | CH | $-N(CH_3)_2$ | 1,5736 |
| 26 | 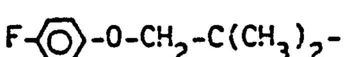 | CH | $-N(CH_3)_2$ | 1,5658 |
| 27 | 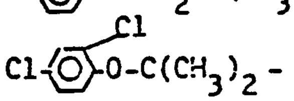 | CH | $-N(CH_3)_2$ | öl |

**Herstellung eines Folgeproduktes**

**Beispiel a**

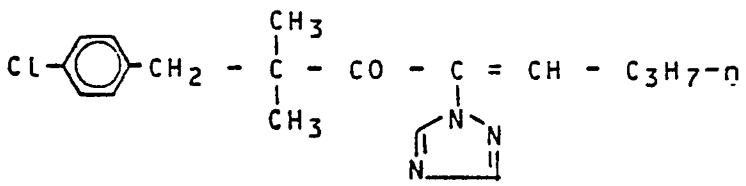

49 9g (0 15 Mol) 1-(4-Chlorphenyl)-2,2-dimethyl-5-dimethyl-amino-4-(1,2,4-triazol-1-yl)-4-penten-3-on (Bsp.1) werden in 750 ml Ether gelöst und bei -20°C tropfenweise mit einer Lösung von 33,9g (0,23 Mol) n-Propylmagnesiumbromid in 100ml Ether versetzt. Man läßt 1,5 Stunden nachrühren, wobei sich das Reaktionsgemisch auf Raumtemperatur erwärmt. Mit verdünnter Salzsäure wird das Reaktionsgemisch auf einen pH-Wert von 7 bis 8 eingestellt. Danach wird die organische Phase abgetrennt, mit Wasser gewaschen, über Natriumsulfat getrocknet und eingeengt. Der Rückstand wird säulenchromatrographisch (Füllmittel Kieselgel; Laufmittel Essigester/Cyclohexan=3:1) gereinigt. Man erhält 30,1 g (60,5 % der Theorie) 1-(4-Chlorphenyl)-2,2-dimethyl-4-(1,2,4-triazol-1-yl)-4-octen-3-on vom Brechungsindex $n_D^{20}=1{,}5429$.

**Beispiel b**

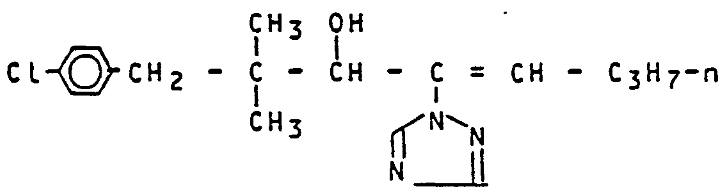

7g (0,021 Mol) 1-(4-Chlorphenyl)-2,2-dimethyl-4-(1,2,4-triazol-1-yl)-4-octen-3-on (Beispiel a) werden in 100 ml Methanol gelöst und bei-10°C tropfenweise mit einer Lösung von 0,21 g (0,006 Mol) Natriumborhydrid in 5ml Eiswasser versetzt. Man läßt 1,5 Stunden bei 0°C nachrühren und stellt dann das Reaktionsgemisch mit verdünnter Salzsäure auf einen pH-Wert von 6 bis 7. Das Reaktionsgemisch wird durch Abdestillation des Lösungsmittels im Vakuum eingeengt. Der Rückstand wird in Chloroform aufgenommen, mit Wasser gewaschen, über Natriumsulfat getrocknet und eingeengt. Man erhält 6,5 g (92,9 % der Theorie) 1-(4-chlorphenyl)-2,2-di-methyl-4-(1,2,4-triazol-1-yl)-4-octen-3-ol vom Brechungsindex $n_D^{20} = 1{,}5383$.

**Patentansprüche**

1) Azolyl-ketoenamine der allgemeinen Formel

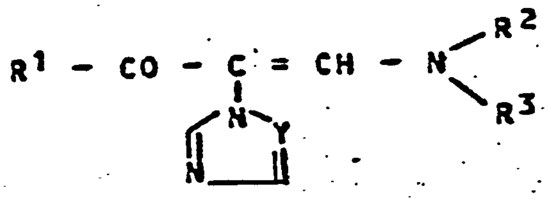 (I)

in welcher stehen

$R^1$ für geradkettiges oder verzweigtes Alkyl mit 1 bis 10 Kohlenstoffatomen; für geradkettiges oder verzweigtes Halogenalkyl mit 1 bis 4 Kohlenstoffund 1 bis 5 gleichen oder verschiedenen Halogenatomen, wie Fluor-, Chlor- und Bromatomen; für gegebenenfalls einfach oder mehrfach, gleich oder verschieden substituiertes Phenyl, wobei als Substituenten genannt seien. Halogen,

Alkyl mit 1 bis 4 Kohlenstoffatomen, Alkoxy und Alkylthio mit jeweils 1 bis 4 Kohlenstoffatomen, Alkyl und Dialkylamino mit jeweils 1 bis 4 Kohlenstoffatomen in jedem Alkylteil, ferner Halogenalkyl, Halogenalkoxy und Halogenalkylthio mit jeweils 1 bis 4 Kohlenstoffatomen und 1 bis 5 gleichen oder verschiedenen Halogenatomen, wie Fluor- und Chloratomen, sowie jeweils gegebenenfalls durch Halogen und Alkyl mit 1 bis 2 Kohlenstoffatomen substituiertes Phenyl und Phenoxy; weiterhin für gegebenenfalls einfach oder mehrfach, gleich oder verschieden durch Alkyl mit 1 bis 4 Kohlenstoffatomen und Halogen substituiertes Cycloalkyl mit 3 bis 7 Kohlenstoffatomen sowie für die Gruppierung

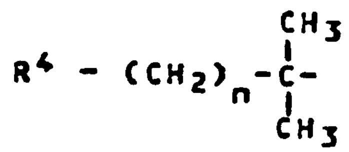

;

$R^4$ für gegebenenfalls einfach oder mehrfach, gleich oder verschieden substituiertes Phenyl, wobei als Substituenten die bei $R^1$ bereits genannten Phenylsubstituenten genannt seien, ferner für geradkettiges oder verzweigtes Alkenyl mit 2 bis 4 Kohlenstoffatomen und Alkinyl mit 3 bis 5 Kohlenstoffatomen, für Cyano sowie für die Gruppierung $-Z-R^5$;

$R^5$ für geradkettiges oder verzweigtes Alkyl mit 1 bis 6 Kohlenstoffatomen; Halogenalkyl mit 1 bis 4 Kohlenstoffatomen und 1 bis 5 gleichen oder verschiedenen Halogenatomen, wie Fluor- und Chloratomen; sowie für jeweils gegebenenfalls einfach oder mehrfach, gleich oder verschieden substituiertes Phenyl und Phenylalkyl mit 1 bis 2 kohlenstoffatomen im Alkylteil, wobei jeweils als Phenylsubstituenten die bei

$R^1$ bereits genannten Phenylsubstituenten infragekommen;

Z für O, S, SO oder $SO_2$,

n für die Zahlen 0 bis 2

$R^2$ und $R^3$ gemeinsam mit dem N-Atom, an das sie gebunden sind, für jeweils gegebenenfalls einfach bis dreifach. gleich oder verschieden durch Alkyl mit 1 bis 4 Kohlenstoffatomen substituiertes Piperidinyl, Pyrrolidinyl oder Morpholinyl; oder

$R^2$ und $R^3$ sind gleich oder verschieden und stehen auch für Alkyl mit 1 bis 4 Kohlenstoffatomen, wenn $R^1$ für gegebenenfalls substituiertes Cycloalkyl oder die Gruppierung $R^4$-$(CH_2)_n$-$C(CH_3)_2$- steht; und

Y für ein Stickstoffatom oder die CH-Gruppe.

2) Verbindungen der allgemeinen Formel (I) in Anspruch 1, wobei

$R^1$ für geradkettiges oder verzweigtes Alkyl mit 4 bis 7 Kohlenstoffatomen steht; für Chlor-tert.-butyl, Fluor-tert.-butyl, 1,3-Dichlor-2-methyl-2-propyl und 1,3-Difluor-2-methyl-2-propyl steht; ferner für gegebenenfalls einfach bis dreifach, gleich oder verschieden substituiertes Phenyl steht, wobei als Substituenten genannt seien: Fluor, Chlor, Methyl, Isopropyl, tert.-Butyl, Methoxy, Methylthio, Isopropoxy, Trifluormethyl, Trifluormethoxy, Trifluormethylthio, Methylamino, Dimethylamino, gegebenenfalls durch Fluor, Chlor, Methyl substituiertes Phenyl und Phenoxy; außerdem für jeweils gegebenenfalls einfach bis dreifach, gleich oder verschieden durch Methyl, Ethyl, Isopropyl, tert.-Butyl, Chlor oder Brom substituiertes Cyclopropyl, Cyclopentyl und Cyclohexyl steht; sowie für die Gruppierung

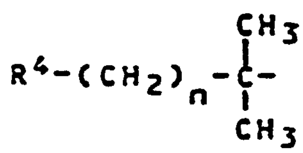

steht;

steht;

$R^4$ für gegebenenfalls einfach bis dreifach, gleich oder verschieden substituiertes Phenyl steht, wobei als Substituenten die bei $R^1$ bereits genannten Phenylsubstituenten genannt seien; ferner für Vinyl, Propargyl, Cyano sowie die Gruppierung$-Z-R^5$ steht;

$R^5$ für geradkettiges oder verzweigtes Alkyl mit 1 bis 4 Kohlenstoffatomen steht, sowie für jeweils gegebenenfalls einfach bis dreifach, gleich oder verschieden substituiertes Phenyl und Benzyl steht, wobei jeweils als Phenylsubstituenten die bei $R^1$ bereits genannten Phenylsubstituenten infragekommen;

$R^2$ und $R^3$ gemeinsam mit dem N-Atom, an das sie gebunden sind, für jeweils gegebenenfalls einfach bis dreifach, gleich oder verschieden durch Methyl, Ethyl und Isopropyl substituiertes piperidinyl pyrrolidinyl oder Morpholinyl stehen; oder

$R^2$ und $R^3$ gleich oder verschieden sind und für Methyl, Ethyl, n-Propyl, i-Propyl oder n-Butyl stehen, wenn $R^1$ für gegebenenfalls substituiertes Cycloalkyl oder die Gruppierung $R^4$-$(CH_2)_n$-$C(CH_3)_2$-steht und

Y, Z und der Index n für die in Anspruch 1 angegebenen Bedeutungen stehen.

3) Verfahren zur Herstellung von Azolyl-ketoenaminen der allgemeinen Formel (I) in Anspruch 1,

dadurch gekennzeichnet, daß man Azolylketone der allgemeinen Formel

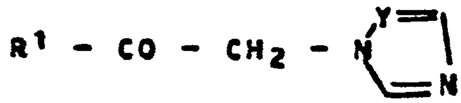

(II)

in welcher
$R^1$ und Y die in Anspruch 1 angegebene Bedeutung haben,
mit Amidacetalen bzw. Aminalestern der Formel

FIG25/30

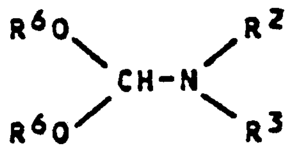

(IIIa)

bzw.

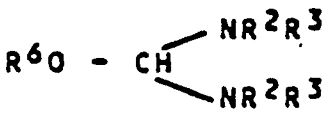

(IIIb)

in welcher
$R^2$ und $R^3$ die in Anspruch 1 angegebene Bedeutung haben
und
$R^6$ für Alkyl mit 1 bis 4 Kohlenstoffatomen steht,
in Gegenwart eines Verdunnungsmittels umsetzt.

4) Verwendung von Azolyl-ketoenaminen der Formel (I) in Anspruch 1 zur Synthese von Azolylvinylketonen und -carbinolen.

**Claims**

1. Azolyi-ketoenamines of the general formula

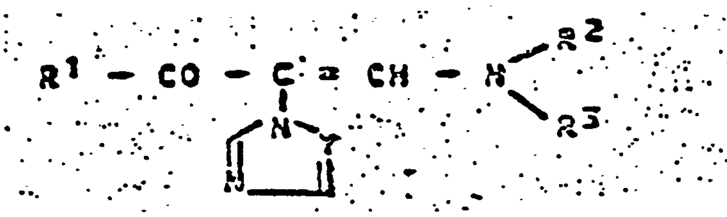

(I)

in which
$R^1$ represents straight-chain or branched alkyl with 1 to 10 carbon atoms; straight-chain or branched halogenoalkyl with 1 to C carbon and 1 to 5 identical or different halogen atoms such as fluorine, chlorine and bromine atoms; phenyl which is optionally mono- or polysubstituted by identical or different substituents, the substituents which may be mentioned being: halogen, alkyl with 1 to 4 carbon atoms, alkoxy and alkylthio with in each case 1 to 4 carbon atoms, alkyl and dialkylamino with in each case 1 to 4 carbon atoms in each alkyl cart, furthermore halogenoalkyl, halogenoalkoxy and halogenoalkylthio with in each case 1 to 4 carbonatoms and 1 to 5 identical or different halogen atoms, such as fluorine and chlorine atoms, and phenyl and phenoxy which are in each case optionally substituted by halogen and alkyl with - to 2 carbon atoms; in addition cycloalkyl which has 3 to 7 carbon atoms and is optionally mono- or polysubstituted by identical or different substituents from the grouo comprising alkyl with 1 to 4 carbon atoms and halogen, and the grouping

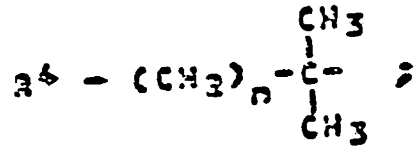

$R^4$ represents phenyl which is optionally mono- or polysubstituted by identical or different substituents, the substituents which may be mentioned being the phenyl substituents already mentioned under $R^1$, furthermore straight-chain or branched alkenyl with 2 to 4 carbon atoms and alkinyl with 3 to 5 carbon atoms, cyano and the grouping -Z-$R^5$;

$R^5$ represents straight-chain or branched alkyl with 1 to 6 carbon atoms; halogenoalkyl with 1 to 4 carbon atoms and 1 to 5 identical or different halogen atoms, such as fluorine and chlorine atoms; and phenyl and phenylalkyl with 1 to 2 carbon atoms in the alkyl part, which are in each case optionally mono- or polysubstituted by identical or different substituents, possible phenyl substituents in each case being the phenyl substituents already mentioned under $R^1$;

Z represents 0, S, SO or $SO_2$,

n represents the numbers 0 to 2,

$R^2$ and $R^3$, together with the N atom to which they are bonded, represent piperidinyl, pyrrolidinyl or morpholinyl which are in each case optionally mono- to tri-substituted by identical or different alkyl radicals with 1 to 4 carbon atoms: or

$R^2$ and $R^3$ are identical or different and also represent alkyl with 1 to 4 carbon atoms when $R^1$ represents optionally substituted cycloalkyl or the grouping $R^4$-$(CH_2)_n$-$C(CH_3)_2$; and

Y represents a nitrogen atom or the CH group.

2. Compounds of the general formula (I) in Claim 1,

wherein

$R^1$ represents straight-chain or branched alkyl with 4 to 7 carbon atoms represents chloro-tert.-butyl, fluoro-tert.-butyl, 1,3-dichloro-2-methyl-2-propyl and 1,3-difluoro-2-methyl-2-propyl; also represents phenyl wich is optionally mono- to trisubstituted by identical or different substituents, the substituents which may be nentioned being: fluorine, chlorine, methyl, isopropyl, tert.-butyl, methoxy, methylthio, isopropoxy, trifluoromethyl, trifluoromethoxy, trifluoromethylthio, methylamino, dimethylamino, and phenyl and phenoxy which are optionally substituted by fluorine, chlorine or methyl; in addition represents cyclopropyl, cyclopentyl and cyclohexyl which are in each case optionally mono- to trisubstituted by identical or different substituents from the group comprising methyl, ethyl, isopropyl, tert.-butyl, chlorine and bromine; and represents the grouping

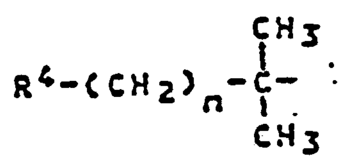

$R^4$ represents phenyl which is optionally mono- to trisubstituted by identical or different substituents, the substituents which may being mentioned being the phenyl substituents already mentioned under $R^1$; and also represents Vinyl, propargyl cyano and the grouping -Z-$R^5$;

$R^5$ represents straight-chain or branched alkyl with 1 to 4 carbon atoms, and represents phenyl and benzyl which are in each case optionally mono- to trisubstituted by identical or different substituents, possible phenyl substituents in each case being the pheny1 substituents already mentioned under

RI;

$R^2$ and $R^3$, together with the N atom to which they are bonded, represent piperidinyl, pyrrolidinyl or morpholinyl, which case optionally mono- to trisubstitued by identical or different substituents from the group comprising methyl, ethyl and isopropyl; or

$R^2$ and $R^3$ are identical or different and represent methyl, ethyl, n-propyl, i-propyl or n-butyl, when $R^1$ represents optionally substituted cycloalkyl or the grouping $R^4$-$(CH_2)_n$-$C(CH_3)_2$

and

Y, Z and the index n represent the meanings given in Claim 1.

3. Process for the preparation of azolyl-ketoenamines of the general formula (I) in Claim 1, characterised in that azolyl ketones of the general formula

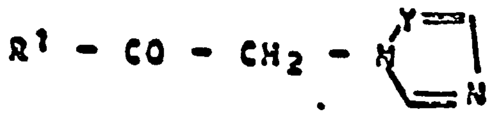

(II)

in which
$R^1$ and Y have the meaning given in Claim 1, are reacted with amide acetals or aminal esters of the formula

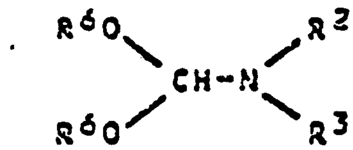

(IIIa)

or

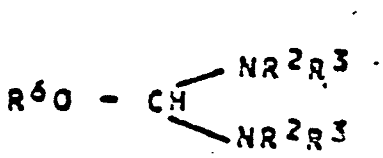

(IIIb)

in which
$R^2$ and $R^3$ have the meaning given in Claim 1 and
$R^6$ represents alkyl with 1 to 4 carbon atoms, in the presence of a diluent.

4. Use of azolyl-ketoenamines of the formula (I) in Claim 1 for the synthesis of azolylvinyl ketones and carbinols.

**Revendications**

1. Azolyl-cétoènamines de formule générale:

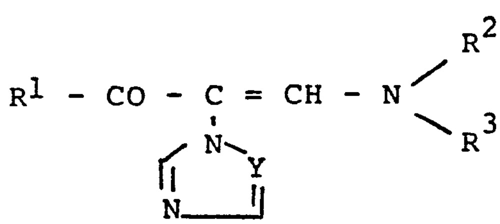

(I)

dans laquelle
$R^1$ représente un alcoyle à chaîne droite ou ramifiée ayant 1 à 10 atomes de carbone, un halogénoalcoyle à chaîne droite ou ramifiée ayant 1 à 4 atomes de carbone et 1 à 5 atomes d'halogène identiques ou différents, comme des atomes de fluor, de chlore et de brome, un phényle éventuellement substitué une ou plusieurs fois, de manière identique ou différente, en citant comme substituants: halogène, alcoyle ayant 1 à 4 atomes de carbone, alcoxy et alcoylthio ayant chacun 1 à 4 atomes de carbone, alcoylet dialcoylamino ayant chacun 1 à 4 atomes de carbone dans chaque portion alcoyle, en outre halogénoalcoyle, halogénoalcoxy et halogénoalcoylthio ayant chacun 1 à 4 atomes de carbone et 1 à 5 atomes d'halogène identiques ou différents, comme des atomes de fluor et de chlore, de même que phényle et phénoxy éventuellement substitués chacun par de l'halogène et par un alcoyle ayant 1 à 2 atomes de carbone, en outre un cycloalcoyle ayant 3 à 7 atomes de carbone et éventuellement substitué une ou plusieurs fois, de manière identique ou différente, par un alcoyle ayant 1 à 4 atomes de carbone et par de l'halogène, de même que le groupement:

$$R^4-(CH_2)_n-\underset{CH_3}{\overset{CH_3}{\underset{|}{\overset{|}{C}}}}-$$

$R^4$ un phényle éventuellement substitué une ou plusieur fois, de manière identique ou différente, en mentionnant comme substituants les substituants du phényle déjà cités pour $R^1$, en outre un alcenyle ayant 2 à 4 atomes de carbone et alcinyle ayant 3 à 5 atomes de carbone à chaîne droite ou ramifiée, un cyano de même que le groupement $-Z-R^5$,

$R^5$ un alcoyle à chaîne droite ou ramifiée ayant 1 à 6 atomes de carbone, un halogénoalcoyle ayant 1 à 4 atomes de carbone et 1 à 5 atomes d'halogène identiques ou différents, tels que des atomes de fluor et de chlore, de même qu'un phényle et phénylalcoyle ayant 1 à 2 atomes de carbone dans la portion alcoyle et éventuellement substitués chacun une ou plusieurs fois, de manière identique ou différente, en envisageant chaque foiscomme substituants du phényle les substituants du phényle déjà cités pour $R^1$,

Z O, S, SO ou $SO_2$,

n les nombres 0 à 2,

$R^2$ et $R^3$, en commun avec l'atome d'azote auquel ils sont attachés, un pipéridinyle, pyrrolidinyle ou morpholinyle éventuellement substitués chacun par un alcoyle ayant 1 à 4 atomes de carbone,

ou bien

$R^2$ et $R^3$ sont identiques ou différents et représentent aussi un alcoyle ayant 1 à 4 atomes de carbone lorsque $R^1$ représente un cycloalcoyle éventuellement substitué ou le groupement $R^4-(CH_2)_n-C(CH_3)_2$

et

Y un atome d'azote ou le groupe CH.

2. Composés de formule générale (I) selon la revendication 1, dans lesquels

$R^1$ représente un alcoyle à chaîne droite ou ramifiée ayant; à 7 atomes de carbone, un chloro-t-butyle, 1-fluoro-t-butyle, 1,3-dichloro-2-méthyl-2-propyle et 1,3-difluoro-2-méthyl-2-propyle, en outre un phényle éventuellement substitué une à trois fois, de manière identique ou différente, en citant en tant que substituants: fluor, chlore, méthyle, isopropyle, t-butyle, méthoxy, méthylthio, isopropoxy, trifluorométhyle, trifluoromethoxy, trifluorométhylthio, méthylamino, diméthylamino, un phényle et un phénoxy éventuellement substitués par du fluor, du chlore, un méthyle, en outre un cyclopropyle, cyclopentyle et cyclohexyle éventuellement substitués chacun une à trois fois, de manière identique ou différente, par un méthyle, éthyle, isopropyle, t-butyle, du chlore ou du brome, ainsi que le groupement

$$R^4-(CH_2)_n-\underset{CH_3}{\overset{CH_3}{\underset{|}{\overset{|}{C}}}}-$$

$R^4$ un phényle éventuellement substitué une à trois fois, de manière identique ou différente, en mentionnant comme substituants les substituants du phényle déjà cités pour $R^1$, en outre un vinyle, propargyle, cyano et aussi le groupement $-Z-R^5$,

$R^5$ un alcoyle à chaîne droite ou ramifiée ayant 1 à 4 atomes de carbone, de même qu'un phényle et un benzyle éventuellement substitués chacun une à trois fois, de manière identique ou différente, en envisageant en tant que substituants du phényle les substituants du phényle déjà cités pour $R^1$,

$R^2$ et $R^3$, en commun avec l'atome d'azote auquel ils sont attachés, représentent un pipéridinyle, pyrrolidinyle ou morpholinyle éventuellement substitués chacun une à trois fois, de manière identique ou différente, par un méthyle, éthyle et isopropyle, ou

$R^2$ et $R^3$ sont identiques ou différents et représentent un methyle, éthyle, n-propyle, i-propyle ou n-butyle lorsque $R^1$ représente un cycloalcoyle éventuellement substitué ou le groupement $R^4-(CH_2)_n-C(CH_3)_2$-et

Y, Z et l'indice n ont les significations indiquées à la revendication 1.

3. Procédé de fabrication d'azolyl-cétoènamines de formule générale (I) selon la revendication 1, caractérisé en ce qu'on fait réagir des azolylcétones de formule générale:

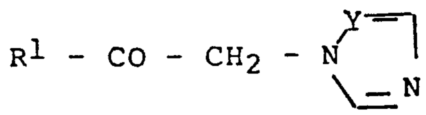

(II)

dans laquelle
$R^1$ et Y ont la signification indiquée à la revendication 1,
avec des amidacétals ou des aminalesters de formules:

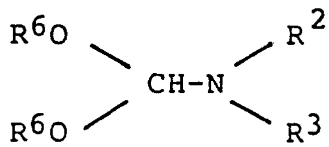

(IIIa)

ou

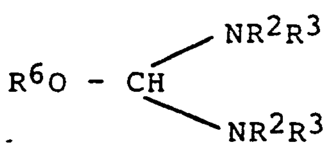

(IIIb)

dans lesquelles
$R^2$ et $R^3$ ont la signification indiquée à la revendication 1 et
$R^6$ est un alcoyle ayant 1 à 4 atomes de carbone, en présence d'un diluant.

4. Utilisation des azolyl-cétoènamines de formule (I) selon la revendication 1 pour la synthèse d'azolylvinylcétones et -carbinols.